(19) Europäisches Patentamt

European Patent Office

Office européen des brevets

(11) **EP 3 899 906 B1**

(12) **FASCICULE DE BREVET EUROPEEN**

(45) Date de publication et mention de la délivrance du brevet:
**04.12.2024 Bulletin 2024/49**

(21) Numéro de dépôt: **19828274.1**

(22) Date de dépôt: **20.12.2019**

(51) Classification Internationale des Brevets (IPC):
**G08G 3/00** *(2006.01)*     **G06N 20/00** *(2019.01)*
**G06N 5/00** *(2023.01)*     **G06F 16/35** *(2019.01)*
**G06F 16/45** *(2019.01)*     **G16H 50/20** *(2018.01)*
**G06N 3/08** *(2023.01)*

(52) Classification Coopérative des Brevets (CPC):
**G08G 3/00; G06N 3/08; G16H 50/20**

(86) Numéro de dépôt international:
**PCT/EP2019/086605**

(87) Numéro de publication internationale:
**WO 2020/127923 (25.06.2020 Gazette 2020/26)**

(54) **PROCÉDÉ D'ÉCHANGES DE DONNÉES ENTRE DES ENTITÉS**

VERFAHREN ZUM DATENAUSTAUSCH ZWISCHEN EINHEITEN

METHOD FOR EXCHANGING DATA BETWEEN ENTITIES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.12.2018 FR 1873867**

(43) Date de publication de la demande:
**27.10.2021 Bulletin 2021/43**

(73) Titulaire: **THALES**
**92400 Courbevoie (FR)**

(72) Inventeurs:
• **GRAH, Simon**
**91767 Palaiseau (FR)**
• **THOUVENOT, Vincent**
**92622 Gennevilliers (FR)**
• **BOETTO, Christophe**
**91767 Palaiseau (FR)**

(74) Mandataire: **Lavoix**
**2, place d'Estienne d'Orves**
**75441 Paris Cedex 09 (FR)**

(56) Documents cités:
EP-A1- 3 291 206     WO-A1-2004/053814
CN-A- 107 797 149     US-A1- 2010 052 948
US-B1- 9 569 959

• **PAPERNOT NICOLAS ET AL: "The Limitations of Deep Learning in Adversarial Settings", 2016 IEEE EUROPEAN SYMPOSIUM ON SECURITY AND PRIVACY (EUROS&P), IEEE, 21 March 2016 (2016-03-21), pages 372 - 387, XP032899541, ISBN: 978-1-5090-1751-5, [retrieved on 20160509], DOI: 10.1109/EUROSP.2016.36**
• **BATTISTA BIGGIO ET AL: "Security Evaluation of Pattern Classifiers under Attack", ARXIV.ORG, CORNELL UNIVERSITY LIBRARY, 201 OLIN LIBRARY CORNELL UNIVERSITY ITHACA, NY 14853, 2 September 2017 (2017-09-02), XP080817905, DOI: 10.1109/TKDE.2013.57**
• **POTHITOS MICHAIL ET AL: "Multi-sensor image fusion and target classification for improved maritime domain awareness", 2016 19TH INTERNATIONAL CONFERENCE ON INFORMATION FUSION (FUSION), ISIF, 5 July 2016 (2016-07-05), pages 1170 - 1177, XP032935141**

**Description**

La présente invention concerne un procédé d'échanges de données entre des entités. La présente invention se rapporte aussi à un produit programme d'ordinateur associé.

Dans le domaine naval, les échanges de données entre un navire et des opérateurs à distance permettent aux opérateurs de contrôler la cohérence du trajet du navire en fonction de la nature du navire. Pour cela, les opérateurs sont, par exemple, amenés à comparer le trajet du navire avec des trajets déjà effectués par des navires de même nature.

Lorsqu'un opérateur détecte qu'un navire effectue un trajet non-cohérent, il est alors susceptible de mettre en place une surveillance plus approfondie du navire afin de mieux comprendre la cause du trajet inhabituel et, éventuellement, d'identifier des activités illicites.

Néanmoins, la mise en place d'une telle surveillance est chronophage et la fiabilité de ses résultats n'est pas garantie.

Le brevet US9569959B1 concerne un procédé de surveillance du trafic dans une zone. Le procédé consiste à recevoir, au niveau de l'électronique de traitement, des données concernant un véhicule actuellement détecté dans la zone, les données comprenant une ou plusieurs caractéristiques du véhicule actuellement détecté. Le procédé consiste également à comparer, par l'électronique de traitement, la ou les caractéristiques du véhicule actuellement détecté à un modèle, le modèle étant basé sur une ou plusieurs caractéristiques de véhicules détectés précédemment dans la zone. Le procédé consiste en outre à déterminer, par l'électronique de traitement, la ou les caractéristiques du véhicule actuellement détecté comme étant anormales sur la base de la comparaison. Le procédé consiste en outre à fournir, par l'électronique de traitement, une indication du véhicule actuellement détecté présentant une ou plusieurs caractéristiques anormales.

Il existe donc un besoin pour un procédé d'échange de données entre des entités qui permettent de mieux évaluer les données échangées entre les entités.

Pour cela, la présente description porte sur un procédé d'échange selon la revendication 1.

Suivant des modes de réalisation particuliers, le procédé d'échange est selon l'une des revendications 2 à 9.

La présente description se rapporte également à un produit programme d'ordinateur selon la revendication 10.

La présente description concerne aussi un support lisible d'informations sur lequel est mémorisé un produit programme d'ordinateur tel que décrit précédemment.

D'autres caractéristiques et avantages de l'invention apparaîtront à la lecture de la description qui suit de modes de réalisation de l'invention, donnés à titre d'exemple uniquement et en référence aux dessins qui sont :

[Fig 1] la figure 1 est une représentation schématique d'un exemple de plusieurs entités échangeant des données,
[Fig 2] la figure 2 est une représentation schématique d'un exemple d'un calculateur de l'une des entités de la figure 1,
[Fig 3] la figure 3 est un organigramme d'un exemple de mise en oeuvre d'un procédé d'échange de données,
[Fig 4] la figure 4 est une représentation schématique d'un exemple expliquant la contribution de chaque variable descriptive d'un vecteur descripteur à l'obtention d'une classe prédite par un modèle de prédiction, et
[Fig 5] la figure 5 est une représentation schématique d'un exemple d'un premier élément et de deuxièmes éléments déterminés au cours de la mise en oeuvre d'un procédé d'échange de données.

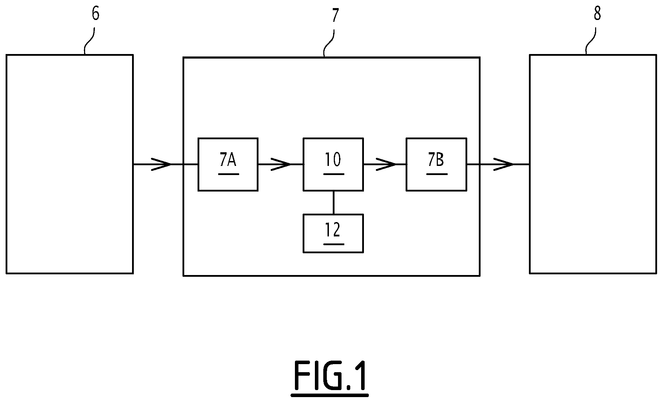

Une première entité 6, une deuxième entité 7 et une troisième entité 8 sont illustrés par la figure 1.

La première entité 6 est, typiquement, une entité propre à collecter des données relatives à un premier élément. Les données collectées forment le vecteur descripteur du premier élément. Le vecteur descripteur comprend un ensemble de variables descriptives.

La première entité 6 est, par exemple, un dispositif ou une personne physique.

Par exemple, dans le domaine naval, la première entité 6 est un navire propre à délivrer des messages AIS (de l'anglais *Automatic Identification System,* traduit en français par *Système d'identification automatique)* à une fréquence d'émission dépendant de caractéristiques du navire (par exemple un bateau au port émettra un message à une fréquence moins élevé qu'un navire à flot). Le premier élément est alors un message AIS délivré par le navire.

Dans le domaine de la maintenance, la première entité 6 est, par exemple, un calculateur ou un opérateur. Le premier élément est, par exemple, un équipement à surveiller.

Dans le domaine médical, la première entité 6 est, par exemple, une unité de mesure, un calculateur ou une personne physique, tel qu'un médecin. Le premier élément est, par exemple, une tumeur à analyser.

La première entité 6 est propre à fournir le vecteur descripteur du premier élément à la deuxième entité 7, par exemple, via une unité d'émission. La transmission de données entre la première entité 6 et la deuxième entité 7 est effectuée directement ou indirectement par l'intermédiaire d'entités intermédiaires.

La deuxième entité 7 est, typiquement, une entité configurée pour analyser le premier élément en fonction du vecteur descripteur du premier élément. La deuxième entité 7 est, avantageusement, propre à transmettre le résultat de l'analyse du premier élément à la troisième entité 8. La transmission de données entre la deuxième entité 7 et la troisième entité 8 est effectuée directement ou indirectement par l'intermédiaire d'entités intermédiaires.

Comme illustré par la figure 1, la deuxième entité 7 comprend une unité de réception 7A, une unité d'émission 7B, un calculateur 10 et un produit programme d'ordinateur 12.

L'unité de réception 7A et l'unité d'émission 7B forment, par exemple, un système antennaire.

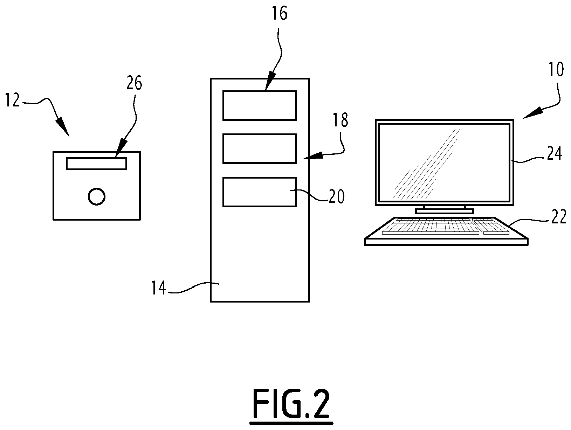

Le calculateur 10 et le produit programme d'ordinateur 12 sont illustrés plus en détails par la figure 2.

Le calculateur 10, est de préférence, un ordinateur.

Plus généralement, le calculateur 10 est un calculateur électronique propre à manipuler et/ou transformer des données représentées comme des quantités électroniques ou physiques dans des registres de calculateur 10 et/ou des mémoires en d'autres données similaires correspondant à des données physiques dans des mémoires, des registres ou d'autres types de dispositifs d'affichage, de transmission ou de mémorisation.

Le calculateur 10 est en interaction avec le produit programme d'ordinateur 12.

Comme illustré par la figure 2, le calculateur 10 comporte un processeur 14 comprenant une unité de traitement de données 16, des mémoires 18 et un lecteur 20 de support d'informations. Dans l'exemple illustré par la figure 2, le calculateur 10 comprend un clavier 22 et une unité d'affichage 24.

Le produit programme d'ordinateur 12 comporte un support d'informations 26.

Le support d'information 26 est un support lisible par le calculateur 10, usuellement par l'unité de traitement de données 16. Le support lisible d'informations 26 est un médium adapté à mémoriser des instructions électroniques et capable d'être couplé à un bus d'un système informatique.

A titre d'exemple, le support d'informations 26 est une disquette ou disque souple (de la dénomination anglaise « *Floppy disc* »), un disque optique, un CD-ROM, un disque magnéto-optique, une mémoire ROM, une mémoire RAM, une mémoire EPROM, une mémoire EEPROM, une carte magnétique ou une carte optique.

Sur le support d'informations 26 est mémorisé le programme d'ordinateur 12 comprenant des instructions de programme.

Le programme d'ordinateur 12 est chargeable sur l'unité de traitement de données 16 et est adapté pour entraîner la mise en oeuvre d'étapes d'un procédé d'échange de données lorsque le programme d'ordinateur 12 est mis en oeuvre sur l'unité de traitement 16 du calculateur 10.

La troisième entité 8 est, typiquement, une entité propre à recevoir les données envoyées par la deuxième entité 7 et à prendre des décisions relatives au premier élément, en fonction des données reçues.

Par exemple, la troisième entité 8 est une personne physique. En variante, la troisième entité 8 est un calculateur comprenant une mémoire dans lequel les données reçues sont mémorisées.

Dans un exemple, la première entité 6 et la troisième entité 8 sont identiques, c'est-à-dire que la première et la troisième entité 8 forme une seule et même entité.

Dans encore un autre exemple, la première entité 6, la deuxième entité 7 et/ou la troisième entité 8 sont identiques.

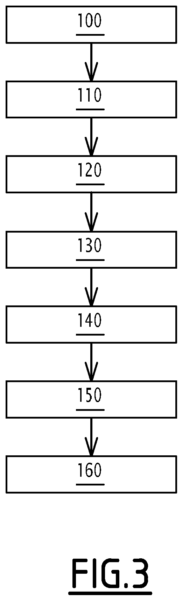

Un exemple d'un procédé d'échange de données entre des entités va maintenant être décrit en référence à la figure 3.

Le procédé d'échange de données comprend une étape 100 de fourniture d'un modèle de prédiction configuré pour prédire la classe d'un élément parmi un ensemble de classes en fonction d'un vecteur descripteur de l'élément. Chaque vecteur descripteur comprend un ensemble de variables descriptives.

Le modèle de prédiction est, par exemple, obtenu par entraînement d'un réseau de neurones selon une base d'entraînement comprenant des vecteurs descripteurs d'éléments et des classes, chaque vecteur descripteur étant associé à une classe.

De manière générale, le modèle de prédiction est configuré pour déterminer une probabilité et pour retourner en sortie une classe correspondant à un intervalle de probabilité.

Dans le domaine naval, les éléments sont, par exemple, des messages AIS. Le vecteur descripteur de chaque message AIS comprend, par exemple, une ou plusieurs des variables descriptives suivantes :

- la vitesse du navire,
- le cap du navire,
- la latitude du navire,
- la longitude du navire,
- le type du navire (cargo, navire pétrolier, etc),
- le statut de navigation du navire (en marche, amarré, etc),
- les données temporelles du message, telles que la date et l'heure de délivrance du message AIS, et
- des données relatives à la nature du message AIS telles que la destination du message AIS.

Dans l'exemple des messages AIS, le modèle de prédiction est configuré pour classifier chaque élément dans des classes relatives à la nature du navire dont est issu le message AIS. Par exemple, le modèle de prédiction est configuré pour prédire deux classes : la classe « cargo » (le navire émetteur est considéré être un cargo) tant que la probabilité que le navire soit un cargo est supérieure à une valeur prédéterminée et la classe « non-cargo » (le navire émetteur est considéré être un bateau différent d'un cargo) lorsque la probabilité que le navire soit un cargo est inférieure à la valeur prédéterminée. Alternativement, le modèle de prédiction est configuré pour prédire plusieurs classes de navires, par exemple, « cargo », « navire pétrolier », « navire de pêche », etc.

En variante, dans le domaine de la maintenance, les éléments sont, par exemple, des équipements à surveiller. Le vecteur descripteur de chaque équipement comprend, par exemple, une ou plusieurs des variables descriptives suivantes :

- le modèle de l'équipement,
- la date de mise en service de l'équipement,
- des données relatives à l'environnement de l'équipement (température, par exemple), et
- la date du dernier défaut détecté sur un ou chaque composant de l'équipement.

Dans l'exemple des équipements à surveiller, le modèle de prédiction est configuré pour classifier chaque élément dans des classes relatives à l'opportunité d'effectuer une action de maintenance sur l'équipement. Par exemple, le modèle de prédiction est configuré pour prédire deux classes : la classe « action de maintenance requise » et la classe « action de maintenance non requise ».

Encore en variante, dans le domaine médical, les éléments sont, par exemple, des tumeurs à analyser. Le vecteur descripteur de chaque tumeur comprend, par exemple, une ou plusieurs des variables descriptives suivantes :

- le rayon de la tumeur,
- la densité de la tumeur,
- la dureté de la tumeur,
- la circonférence de la tumeur, et
- le périmètre de la tumeur.

Dans l'exemple des tumeurs, le modèle de prédiction est configuré pour classifier chaque élément dans des classes relatives à la probabilité que la tumeur soit cancéreuse ou non. Par exemple, le modèle de prédiction est configuré pour prédire deux classes : la classe « cancéreuse » et la classe « non-cancéreuse ».

Le procédé d'échange de données comprend une étape 110 de réception, par l'unité de réception 7A de la deuxième entité 7, d'un vecteur descripteur d'un premier élément. Le vecteur descripteur du premier élément est avantageusement en provenance de la première entité 6.

Avantageusement, au moins une variable descriptive du vecteur descripteur est issue de mesures effectuées par au moins un capteur, que ce soient des mesures de l'environnement du premier élément ou des mesures du premier élément lui-même.

Le procédé d'échange de données comprend une étape 120 de prédiction de la classe du premier élément par le modèle de prédiction en fonction du vecteur descripteur du premier élément. L'étape de prédiction 120 est mise en oeuvre par le calculateur 10 en interaction avec le produit programme d'ordinateur 12, c'est-à-dire est mise en oeuvre par ordinateur.

Par exemple, dans le domaine naval, lorsque le premier élément est un message AIS, la classe du premier élément est prédite parmi la classe « cargo » et la classe « non-cargo ».

Par exemple, dans le domaine de la maintenance, lorsque le premier élément est un équipement à surveiller, la classe du premier élément est prédite parmi la classe « action de maintenance requise » et la classe « action de maintenance non requise ».

Par exemple, dans le domaine médical, lorsque le premier élément est une tumeur à analyser, la classe du premier élément est prédite parmi la classe « cancéreuse » et la classe « non-cancéreuse ».

Le procédé d'échange de données comprend une étape 130 de détermination, pour l'obtention de la classe du premier élément, de la contribution de chaque variable descriptive du premier élément. En d'autres termes, l'étape 130 vise à déterminer la contribution de chaque variable descriptive dans la sortie du modèle de prédiction. Avantageusement, la somme des contributions déterminées de chaque variable permet d'expliquer la sortie du modèle de prédiction. L'étape de détermination 130 est mise en oeuvre par le calculateur 10 en interaction avec le produit programme d'ordinateur 12, c'est-à-dire est mise en oeuvre par ordinateur.

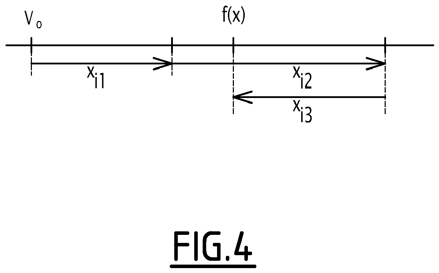

Dans l'exemple illustré par la figure 4, le vecteur descripteur est noté « x », le modèle de prédiction est noté « f » et la classe prédite par le modèle de prédiction est notée « f(x) ». Le vecteur descripteur x comprend trois variables descriptives : $x_{i1}$, $x_{i2}$ et $x_{i3}$. Une valeur de base correspondant, par exemple, à la sortie moyenne du modèle de prédiction est notée « $V_0$ ». Dans cet exemple, la première variable descriptive $x_{i1}$ et la deuxième variable descriptive $x_{i2}$ ont contribué à augmenter la valeur de f(x) par rapport à la valeur de base $V_0$, alors que la troisième variable descriptive $x_{i3}$

a contribué à baisser la valeur de f(x) par rapport à la valeur de base $V_0$. La contribution des première et deuxième variables descriptives $x_{i1}$, $x_{i2}$ est donc positive et plus importante pour la deuxième variable descriptive $x_{i2}$. La contribution de la troisième variable descriptive $x_{i3}$ est négative.

Lorsque le premier élément est un message AIS, la première variable descriptive $x_{i1}$ est, par exemple, la vitesse (1,5 noeuds), la deuxième variable descriptive $x_{i2}$ est, par exemple, le statut de navigation (amarré) et la troisième variable descriptive $x_{i3}$ est, par exemple, la latitude (45°).

Dans un exemple de réalisation, la contribution de chaque variable descriptive est obtenue sur la base d'une méthode utilisée dans le domaine des jeux coopératifs et consistant à répartir un gain entre des joueurs en fonction de leur contribution vis-à-vis du gain. Dans le cas d'espèce, par analogie, le jeu est la tâche de prédiction d'une classe pour un vecteur descripteur x, le gain est la classe prédite f(x) et les joueurs sont les variables descriptibles $x_i$ du vecteur descripteur x. La contribution de chaque variable descriptive du premier élément est alors une valeur de Shapley.

Le procédé d'échange de données comprend une étape 140 de sélection d'un sous-ensemble de variables descriptives du premier élément dont la contribution vérifie un critère de prépondérance relativement aux contributions de l'ensemble des variables descriptives du premier élément. L'étape de sélection 140 est mise en oeuvre par le calculateur 10 en interaction avec le produit programme d'ordinateur 12, c'est-à-dire est mise en oeuvre par ordinateur.

Dans un exemple, le critère de prépondérance stipule que le sous-ensemble sélectionné de variables descriptives comprend les N premières variables descriptives ayant la contribution la plus élevée, N étant un entier naturel prédéterminé.

Dans un autre exemple, le critère de prépondérance stipule que le sous-ensemble sélectionné de variables descriptives comprend :

- la variable descriptive dont la contribution est la plus élevée parmi l'ensemble des variables descriptives, et
- la ou les variables descriptives dont la contribution est supérieure au produit de la contribution la plus élevée par un nombre prédéterminé compris entre zéro et un.

En complément facultatif, des variables descriptives prédéfinies sont fixées et ne peuvent pas être sélectionnées pour faire partie du sous-ensemble de variables descriptives.

Dans l'exemple des messages AIS, les variables descriptives prépondérantes sont, par exemple, la latitude, la vitesse et le statut de navigation.

Dans l'exemple des tumeurs, les variables descriptives prépondérantes sont, par exemple, le rayon et la densité de la tumeur.

Dans l'exemple de la maintenance, une variable descriptive prépondérante est, par exemple, la date du dernier défaut détecté sur un composant de l'équipement.

Le procédé d'échange de données comprend une étape 150 de détermination, le cas échéant, d'au moins un deuxième élément en fonction du vecteur descripteur du premier élément, de la classe prédite pour le premier élément et du sous-ensemble sélectionné de variables descriptives du premier élément. L'étape de détermination est mise en oeuvre par le calculateur 10 en interaction avec le produit programme d'ordinateur 12, c'est-à-dire est mise en oeuvre par ordinateur.

Chaque deuxième élément déterminé forme un exemple contrefactuel. Un exemple contrefactuel est défini comme étant un élément ayant un vecteur descripteur proche du premier élément (exemple de base) mais dont la classe prédite est différente. Un tel exemple contrefactuel permet de mieux comprendre la classe prédite par le modèle de prédiction pour le premier élément. Un exemple contrefactuel est donc un contre-exemple qui a une visée explicative.

Par exemple, dans le cas des messages AIS, pour un premier élément dont la classe prédite est non cargo (prédiction 18%), un exemple contrefactuel pourrait être un deuxième élément avec une seule variable descriptive modifiée par rapport au premier élément (vitesse de 16 noeuds au lieu de 9 noeuds) et dont la classe prédite est cargo (prédiction de 58%).

Dans un autre exemple relatif aux messages AIS, le modèle de prédiction vise à prédire la vitesse d'un navire à un instant donné en fonction des caractéristiques du message AIS. Par exemple, la vitesse prédite est de 12 noeuds. Il est souhaité générer un exemple contrefactuel proche du message AIS initial mais dont la vitesse prédite par le modèle de prédiction est, par exemple, de 21 noeuds. Le deuxième exemple est donc un message AIS présentant des caractéristiques proches du message AIS d'origine mais dont la vitesse prédite par le modèle de prédiction est de 21 noeuds.

Chaque deuxième élément vérifie les conditions suivantes :

- (i) la distance entre le vecteur descripteur du premier élément et le vecteur descripteur du deuxième élément est inférieure à un seuil prédéterminé. Cela permet que le deuxième élément (contre-exemple) soit proche du premier élément (exemple de base).
- (ii) au moins une variable descriptive du deuxième élément à une valeur différente de la variable descriptive correspondante du premier élément et appartient au sous-ensemble sélectionné de variables descriptives du premier élément, et

- (iii) l'image du vecteur descripteur du deuxième élément par le modèle de prédiction est une classe différente de la classe du premier élément.

Avantageusement, chaque deuxième élément vérifie, en outre, au moins l'une des conditions suivantes :

- (iv) chaque variable descriptive du deuxième élément ayant une valeur différente de la variable descriptive correspondante du premier élément appartient au sous-ensemble sélectionné de variables descriptives du premier élément. En d'autres termes, toutes les variables descriptives du deuxième élément qui sont différentes des variables descriptives correspondantes du premier élément sont des variables vérifiant le critère de prépondérance.
- (v) chaque deuxième élément a au plus un nombre M de variables descriptives différentes des variables descriptives correspondantes du premier élément, M étant un entier naturel strictement positif. Cela permet d'obtenir un deuxième élément avec des modifications sur un nombre faible de variables descriptives. En effet, un exemple contrefactuel sera plus facile à interpréter pour un opérateur si les modifications concernent un petit nombre de variables descriptives (même si ces modifications sont importantes), plutôt qu'un grand nombre de variables descriptives avec des modifications faibles.

Avantageusement, le ou les deuxièmes éléments représentant les meilleurs exemples contrefactuels sont les éléments pour lesquels la distance entre le vecteur descripteur du premier élément et le vecteur descripteur du deuxième élément est la plus petite et qui présentent le plus de variables descriptives inchangées par rapport au premier élément.

Avantageusement, le deuxième élément est déterminé en fonction d'un modèle de détermination (tel qu'un réseau de neurones) entraîné selon une base d'entraînement de sorte à respecter toutes ou au moins les contraintes (i), (ii) et (iii) décrites précédemment. La base d'entraînement comprend des vecteurs descripteurs d'éléments et des classes, chaque vecteur descripteur étant associé à une classe. Par exemple, la base d'entraînement est la même base que celle ayant permis d'obtenir le modèle de prédiction. Le fait qu'une base d'entraînement soit utilisée dans la détermination du deuxième exemple permet d'obtenir un deuxième élément réaliste. Dans l'exemple des messages AIS, cela permet d'éviter d'obtenir un deuxième élément ayant une latitude correspondant à un lieu sur terre (ce qui n'est pas réaliste pour un navire) ou dont la vitesse est élevée alors que le navire est amarré.

En variante, le deuxième élément est déterminé sans base d'entraînement. Dans ce cas, les deuxièmes éléments potentiels sont contrôlés par un opérateur ou par des règles, telles que des règles métier, afin d'obtenir un deuxième élément réaliste.

Dans un exemple de mise en oeuvre, la détermination du deuxième élément consiste à minimiser une fonction de coût (1) de la forme suivante :

$$L(z, \lambda \mid x, \tilde{y}) = d(x, z) + \lambda . (f(z) - \tilde{y})^2 \qquad (1)$$

sous la contrainte (2) suivante :

$$|f(z) - \tilde{y}| \leq \varepsilon \qquad (2)$$

Où :

z désigne le vecteur descripteur du deuxième élément (que l'on cherche à déterminer),
x désigne le vecteur descripteur du premier élément,
$\tilde{y}$ désigne une sortie cible du modèle de prédiction, c'est-à-dire une classe ou une probabilité, que l'on souhaite obtenir pour le deuxième élément,
f désigne la fonction appliquée par le modèle de prédiction,
f(z) désigne l'image du vecteur descripteur z du deuxième élément par le modèle de prédiction,
$\lambda$ désigne un facteur de pénalisation, $\lambda$ est un réel,
$\varepsilon$ désigne une marge d'approximation, $\varepsilon$ est un réel, et
d(x,z) désigne la distance entre le vecteur descripteur du premier élément et le vecteur descripteur du deuxième élément.

La distance d est, par exemple, une distance de Manhattan. Une telle distance permet, en effet, de modifier moins de variables descriptives par rapport à une distance euclidienne. Cela permet ainsi de faciliter l'interprétation de l'exemple contrefactuel formé par chaque deuxième élément.

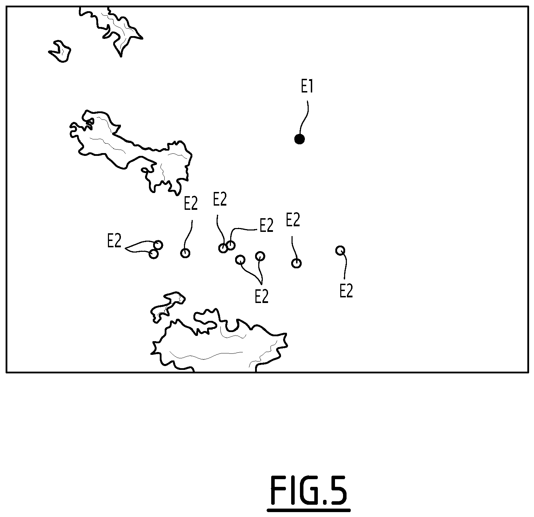

La figure 5 illustre un exemple de deuxièmes éléments E2 déterminés pour un premier élément E1 dans le cas de messages AIS. Dans cet exemple, le premier élément est un message AIS en provenance d'un navire qui a été classifié par le modèle de prédiction comme n'appartenant pas à la classe cargo. Les deuxièmes éléments sont des exemples contrefactuels ayant chacun un vecteur descripteur proche du vecteur descripteur du premier élément mais classifiés par le modèle de prédiction comme appartenant à la classe cargo. Comme illustré par la figure 5, les deuxièmes éléments se trouvant sur un rail, cela permet de comprendre que le premier élément a été classifié comme n'étant pas un cargo car sa route a dévié (latitude notamment).

Le procédé d'échange de données comprend une étape 160 d'envoi, par l'unité d'émission 7B de la deuxième entité 7, à la troisième entité 8, du vecteur descripteur du premier élément, de la classe du premier élément et, le cas échéant, du vecteur descripteur du deuxième élément et de la classe du deuxième élément. Les données sont, par exemple, envoyées à la troisième entité 8 à des fins d'analyse ou de prise de décisions.

Optionnellement, le procédé comprend, en outre, une étape de validation de la classe prédite pour le premier élément lorsqu'aucun deuxième élément ne vérifie l'ensemble des contraintes. La prédiction est donc considérée comme étant fiable.

Optionnellement, les vecteurs descripteurs pour lesquels la classe prédite ne correspond pas à la classe réelle de l'élément (par exemple, un vecteur descripteur classé non-cargo alors que correspondant en réalité à un navire cargo) sont mémorisés dans une base d'anomalies. La base d'anomalies est, par exemple, utilisée pour mettre à jour le modèle de prédiction.

Ainsi, le procédé permet, par la détermination de deuxièmes éléments, de générer des exemples contrefactuels permettant de mieux comprendre les sorties d'un modèle de prédiction. En particulier, les deuxièmes éléments permettent de mieux appréhender l'impact de certaines variables descriptives sur les sorties du modèle. Les contraintes imposées pour la détermination des deuxièmes éléments permettent d'obtenir des exemples contrefactuels facilement interprétables par un opérateur. Plus précisément, la sélection effectuée des variables descriptives du premier élément vérifiant un critère de prépondérance permet de faciliter la détermination de deuxièmes éléments facilement interprétables par l'opérateur.

Dans le domaine naval, un tel procédé permet de mieux comprendre pourquoi un message en provenance d'un navire de type cargo est classé comme étant non-cargo. Cela peut être dû, par exemple à un changement de cap, de latitude ou de vitesse du navire.

Dans le domaine médical, un tel procédé permet au médecin d'être plus précis dans son diagnostic. Notamment, lorsque les éléments sont des tumeurs, si un premier élément est classé non-cancéreuse mais qu'un deuxième élément ayant seulement un rayon plus important que le premier élément est classé cancéreuse, le médecin pourra réévaluer le classement non-cancéreuse qui pourrait être dû à une erreur de mesure du rayon du premier élément.

Dans le domaine de la maintenance, un tel procédé permet de mieux appréhender les actions de maintenance à réaliser sur un équipement à surveiller.

Le procédé permet, également, de déterminer les valeurs d'entrée à modifier pour que la sortie d'un modèle de prédiction atteigne une valeur souhaitée.

Ainsi, le procédé selon l'invention permet d'améliorer la fiabilité des données échangées entre des entités.

L'homme du métier comprendra que le présent procédé s'applique à des domaines variés, autres que ceux décrits précédemment, tels que, par exemple, au domaine de l'avionique. Le présent procédé s'applique, également, à des méthodes non supervisées, par exemple, pour la détection d'anomalies.

**Revendications**

1. Procédé d'échanges de données entre des entités (6, 7, 8), le procédé comprenant les étapes de :

- fourniture d'un modèle de prédiction configuré pour prédire la classe d'un élément parmi un ensemble de classes en fonction d'un vecteur descripteur (x) de l'élément, chaque vecteur descripteur (x) comprenant un ensemble de variables descriptives ($x_i$),
- réception d'un vecteur descripteur (x) d'un premier élément (E1), le vecteur descripteur (x) étant en provenance d'une première entité (6), l'étape de réception étant mise en oeuvre par une unité de réception (7A) d'une deuxième entité (7),
- prédiction de la classe du premier élément (E1) par le modèle de prédiction en fonction du vecteur descripteur (x) du premier élément (E1), l'étape de prédiction étant mise en oeuvre par ordinateur de la deuxième entité,
- détermination, pour l'obtention de la classe du premier élément (E1), de la contribution de chaque variable descriptive ($x_i$) du premier élément (E1), l'étape de détermination étant mise en oeuvre par ordinateur de la deuxième entité,
- sélection d'un sous-ensemble de variables descriptives ($x_i$) du premier élément (E1) dont la contribution vérifie un critère de prépondérance relativement aux contributions de l'ensemble des variables descriptives ($x_i$) du premier élément (E1), l'étape de sélection étant mise en oeuvre par ordinateur de la deuxième entité,
- détermination, le cas échéant, d'au moins un deuxième élément (E2) en fonction du vecteur descripteur (x) du premier élément (E1), de la classe prédite pour le premier élément (E1) et du sous-ensemble sélectionné de variables descriptives ($x_i$) du premier élément (E1), l'étape de détermination étant mise en oeuvre par ordinateur de la deuxième entité, le deuxième élément (E2) vérifiant les conditions suivantes :

- la distance entre le vecteur descripteur (x) du premier élément (E1) et le vecteur descripteur (x) du deuxième élément (E2) est inférieure à un seuil prédéterminé,
- au moins une variable descriptive ($x_i$) du deuxième élément (E2) à une valeur différente de la variable descriptive ($x_i$) correspondante du premier élément (E1) et appartient au sous-ensemble sélectionné de variables descriptives ($x_i$) du premier élément (E1), et
- l'image du vecteur descripteur (x) du deuxième élément (E2) par le modèle de prédiction est une classe différente de la classe du premier élément (E1),

- envoi, par une unité d'émission (7B) de la deuxième entité (7), à une troisième entité (8), du vecteur descripteur (x) du premier élément (E1), de la classe du premier élément (E1) et, le cas échéant, du vecteur descripteur (x) du deuxième élément (E2) et de la classe du deuxième élément (E2).

2. Procédé selon la revendication 1, dans lequel le critère de prépondérance stipule que le sous-ensemble sélectionné de variables descriptives ($x_i$) comprend les N premières variables descriptives ($x_i$) ayant la contribution la plus élevée, N étant un entier naturel prédéterminé.

3. Procédé selon la revendication 1, dans lequel le critère de prépondérance stipule que le sous-ensemble sélectionné de variables descriptives ($x_i$) comprend :

- la variable descriptive ($x_i$) dont la contribution est la plus élevée parmi l'ensemble des variables descriptives ($x_i$), et
- la ou les variables descriptives ($x_i$) dont la contribution est supérieure au produit de la contribution la plus élevée par un nombre prédéterminé compris entre zéro et un.

4. Procédé selon l'une quelconque des revendications 1 à 3, dans lequel la contribution de chaque variable descriptive ($x_i$) du premier élément (E1) est une valeur de Shapley.

5. Procédé selon l'une quelconque des revendications 1 à 4, dans lequel le deuxième élément (E2) est déterminé en fonction d'un modèle de détermination entraîné selon une base d'entraînement, la base d'entraînement comprenant des vecteurs descripteurs d'éléments et des classes, chaque vecteur descripteur (x) étant associé à une classe.

6. Procédé selon l'une quelconque des revendications 1 à 5, dans lequel le deuxième élément (E2) déterminé vérifie, en outre, la condition selon laquelle chaque variable descriptive ($x_i$) du deuxième élément (E2) ayant une valeur différente de la variable descriptive ($x_i$) correspondante du premier élément (E1) appartient au sous-ensemble sélectionné de variables descriptives ($x_i$) du premier élément (E1).

7. Procédé selon l'une quelconque des revendications 1 à 6, dans lequel le procédé comprend une étape de validation de la classe prédite pour le premier élément (E1) lorsqu'aucun deuxième élément (E2) ne vérifie l'ensemble des contraintes.

8. Procédé selon l'une quelconque des revendications 1 à 7, dans lequel au moins une variable descriptive ($x_i$) du vecteur descripteur (x) du premier élément (E1) est issue de mesures effectuées par au moins un capteur.

9. Procédé selon l'une quelconque des revendications 1 à 8, dans lequel la première et la troisième entité (6, 8) sont identiques.

10. Produit programme d'ordinateur comportant un support lisible d'informations, sur lequel est mémorisé un programme d'ordinateur comprenant des instructions de programme, le programme d'ordinateur étant chargeable sur une unité de traitement de données et adapté pour entraîner la mise en oeuvre d'un procédé selon l'une quelconque des revendications 1 à 9 lorsque le programme d'ordinateur est mis en oeuvre sur l'unité de traitement des données.

**Patentansprüche**

1. Verfahren zum Datenaustausch zwischen Entitäten (6, 7, 8), das Verfahren umfassend die folgenden Schritte:

   - Bereitstellen eines Vorhersagemodells, das konfiguriert ist, um die Klasse eines Elements aus einem Satz von Klassen abhängig von einem Deskriptorvektor (x) des Elements vorherzusagen, wobei jeder Deskriptorvektor (x) einen Satz von beschreibenden Variablen ($x_i$) umfasst,
   - Empfangen eines Deskriptorvektors (x) eines ersten Elements (E1), wobei der Deskriptorvektor (x) von einer ersten Entität (6) stammt, wobei der Empfangsschritt von einer Empfangseinheit (7A) einer zweiten Entität (7) implementiert wird,
   - Vorhersagen der Klasse des ersten Elements (E1) durch das Vorhersagemodell abhängig von dem Deskriptorvektor (x) des ersten Elements (E1), wobei der Vorhersageschritt durch den Computer der zweiten Einheit implementiert wird,
   - Bestimmen, um die Klasse des ersten Elements (E1) zu erlangen, des Beitrags von jeder beschreibenden Variablen ($x_i$) des ersten Elements (E1), wobei der Bestimmungsschritt von einem Computer der zweiten Entität implementiert wird,
   - Auswählen eines Teilsatzes von beschreibenden Variablen ($x_i$) des ersten Elements (E1), deren Beitrag ein Übergewichtskriterium in Bezug auf die Beiträge der Gesamtheit der beschreibenden Variablen ($x_i$) des ersten Elements (E1) erfüllt, wobei der Auswahlschritt von einem Computer der zweiten Einheit implementiert wird,
   - gegebenenfalls Bestimmen mindestens eines zweiten Elements (E2) abhängig von dem Deskriptorvektor (x) des ersten Elements (E1), der vorhergesagten Klasse für das erste Element (E1) und dem ausgewählten Teilsatz von beschreibenden Variablen ($x_i$) des ersten Elements (E1), wobei der Bestimmungsschritt von einem Computer der zweiten Entität implementiert wird,

   wobei das zweite Element (E2) die folgenden Bedingungen erfüllt:

   - der Abstand zwischen dem Deskriptorvektor (x) des ersten Elements (E1) und dem Deskriptorvektor (x) des zweiten Elements (E2) ist kleiner als ein vorbestimmter Schwellenwert,
   - mindestens eine beschreibende Variable ($x_i$) des zweiten Elements (E2) weist einen anderen Wert auf als die entsprechende beschreibende Variable ($x_i$) des ersten Elements (E1) und gehört zu dem ausgewählten Teilsatz beschreibender Variablen ($x_i$) des ersten Elements (E1), und
   - das Bild des Deskriptorvektors (x) des zweiten Elements (E2) durch das Vorhersagemodell ist eine Klasse, die sich von der Klasse des ersten Elements (E1) unterscheidet,
   - Senden, durch eine Emissionseinheit (7B) der zweiten Entität (7) an eine dritte Entität (8), des Deskriptorvektors (x) des ersten Elements (E1), der Klasse des ersten Elements (E1) und gegebenenfalls des Deskriptorvektors (x) des zweiten Elements (E2) und der Klasse des zweiten Elements (E2).

2. Verfahren nach Anspruch 1, wobei das Übergewichtskriterium besagt, dass der ausgewählte Teilsatz der beschreibenden Variablen ($x_i$) die ersten N beschreibenden Variablen ($x_i$) mit dem höchsten Beitrag umfasst, wobei N eine vorbestimmte natürliche Zahl ist.

3. Verfahren nach Anspruch 1, wobei das Übergewichtskriterium besagt, dass der ausgewählte Teilsatz der beschreibenden Variablen ($x_i$) Folgendes umfasst:

   - die beschreibende Variable ($x_i$), deren Beitrag von allen beschreibenden Variablen ($x_i$) am höchsten ist, und
   - die beschreibende(n) Variable(n) ($x_i$), deren Beitrag größer ist als das Produkt aus dem höchsten Beitrag und einer vorbestimmten Zahl zwischen null und eins.

4. Verfahren nach einem der Ansprüche 1 bis 3, wobei der Beitrag von jeder beschreibenden Variablen ($x_i$) des ersten Elements (E1) ein Shapley-Wert ist.

5. Verfahren nach einem der Ansprüche 1 bis 4, wobei das zweite Element (E2) abhängig von einem Bestimmungsmodell bestimmt wird, das gemäß einer Trainingsbasis trainiert wird, die Trainingsbasis umfassend Deskriptorvektoren für Elemente und Klassen, wobei jeder Deskriptorvektor (x) mit einer Klasse assoziiert ist.

6. Verfahren nach einem der Ansprüche 1 bis 5, wobei das bestimmte zweite Element (E2) ferner die Bedingung erfüllt, dass jede beschreibende Variable ($x_i$) des zweiten Elements (E2), die einen anderen Wert als die entsprechende beschreibende Variable ($x_i$) des ersten Elements (E1) aufweist, zu dem ausgewählten Teilsatz von beschreibenden

Variablen ($x_i$) des ersten Elements (E1) gehört.

7. Verfahren nach einem der Ansprüche 1 bis 6, wobei das Verfahren einen Schritt umfasst, in dem die vorhergesagte Klasse für das erste Element (E1) validiert wird, wenn kein zweites Element (E2) alle Einschränkungen erfüllt.

8. Verfahren nach einem der Ansprüche 1 bis 7, wobei mindestens eine beschreibende Variable ($x_i$) des Deskriptorvektors (x) des ersten Elements (E1) aus Messungen stammt, die von mindestens einem Sensor durchgeführt werden.

9. Verfahren nach einem der Ansprüche 1 bis 8, wobei die erste und die dritte Entität (6, 8) identisch sind.

10. Computerprogrammprodukt, umfassend einen lesbaren Informationsträger, auf dem ein Computerprogramm gespeichert ist, umfassend Programmanweisungen, wobei das Computerprogramm auf eine Datenverarbeitungseinheit ladungsfähig ist und angepasst ist, um die Implementierung eines Verfahrens nach einem der Ansprüche 1 bis 9 zu bewirken, wenn das Computerprogramm auf der Datenverarbeitungseinheit implementiert wird.

**Claims**

1. A method for exchanging data between entities (6, 7, 8), the method comprising the steps of:

- providing a prediction model configured to predict the class of an element among a set of classes based on a descriptor vector (x) of the element, each descriptor vector (x) comprising a set of descriptive variables ($x_i$),
- receiving a descriptor vector (x) of a first element (E1), the descriptor vector (x) coming from a first entity (6), the receiving step being implemented by a receiving unit (7A) of a second entity (7),
- predicting the class of the first element (E1) by the prediction model based on the descriptor vector (x) of the first element (E1), the prediction step being implemented by computer,
- determining, in order to obtain the class of the first element (E1), the contribution of each descriptive variable ($x_i$) of the first element (E1), the determining step being implemented by computer of the second entity,
- selecting a subset of descriptive variables ($x_i$) of the first element (E1) whose contribution satisfies a preponderance criterion relative to the contributions of the set of descriptive variables ($x_i$) of the first element (E1), the selection step being implemented by computer of the second entity,
- determining, if necessary, at least one second element (E2) based on the descriptor vector (x) of the first element (E1), of the class predicted for the first element (E1) and of the selected subset of descriptive variables ($x_i$) of the first element (E1), the determining step being implemented by computer of the second entity,

the second element (E2) verifying the following conditions:

- the distance between the descriptor vector (x) of the first element (E1) and the descriptor vector (x) of the second element (E2) is less than a predetermined threshold,
- at least one descriptive variable ($x_i$) of the second element (E2) has a value different from the corresponding descriptive variable ($x_i$) of the first element (E1) and belongs to the selected subset of descriptive variables ($x_i$) of the first element (E1), and
- the image of the descriptor vector (x) of the second element (E2) by the prediction model is a class different from the class of the first element (E1),
- sending, by a transmission unit (7B) of the second entity (7), to a third entity (8), the descriptor vector (x) of the first element (E1), the class of the first element (E1) and, where appropriate, the descriptor vector (x) of the second element (E2) and the class of the second element (E2).

2. The method according to claim 1, wherein the preponderance criterion stipulates that the selected subset of descriptive variables ($x_i$) comprises the N first descriptive variables ($x_i$) having the highest contribution, N being a predetermined natural integer.

3. The method according to claim 1, wherein the preponderance criterion stipulates that the selected subset of descriptive variables ($x_i$) comprises:

- the descriptive variable ($x_i$) whose contribution is the highest among all the descriptive variables ($x_i$), and
- the descriptive variable(s) ($x_i$) whose contribution is greater than the product of the highest contribution by a predetermined number between zero and one.

4. The method according to any one of claims 1 to 3, wherein the contribution of each descriptive variable ($x_i$) of the first element (E1) is a Shapley value.

5. The method according to any one of claims 1 to 4, wherein the second element (E2) is determined according to a determination model driven according to a training base, the training base comprising descriptor vectors of elements and classes, each descriptor vector (x) being associated with a class.

6. The method according to any one of claims 1 to 5, wherein the determined second element (E2) further verifies the condition that each descriptive variable ($x_i$) of the second element (E2) having a value different from the corresponding descriptive variable ($x_i$) of the first element (E1) belongs to the selected subset of descriptive variables ($x_i$) of the first element (E1).

7. The method according to any one of claims 1 to 6, wherein the method comprises a step of validating the predicted class for the first element (E1) when no second element (E2) verifies all of the constraints.

8. The method according to any one of claims 1 to 7, wherein at least one descriptive variable ($x_i$) of the descriptor vector (x) of the first element (E1) comes from measurements carried out by at least one sensor.

9. The method according to any one of claims 1 to 8, wherein the first and the third entity (6, 8) are identical.

10. A computer program product comprising a readable information medium, on which a computer program is stored comprising program instructions, the computer program being loadable on a data processing unit and suitable for causing the implementation of a method according to any one of claims 1 to 9 when the computer program is implemented on the data processing unit.

## FIG.1

FIG.2

# FIG.3

$V_o$

$f(x)$

$x_{i1}$

$x_{i2}$

$x_{i3}$

# FIG.4

## FIG.5

**EP 3 899 906 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 9569959 B1